# EUROPÄISCHE PATENTSCHRIFT

(11) **EP 2 278 328 B1**
(45) Veröffentlichungstag und Bekanntmachung des Hinweises auf die Patenterteilung: **24.04.2013**
(21) Anmeldenummer: 10012542.6
(22) Anmeldetag: 15.11.2006
(51) Int. Cl.: G01N 33/36, G01N 21/89

(54) **Verfahren zur Charakterisierung von Effektgarn**
Method for characterising effect yarn
Procédé pour caractériser des fils d'effet

(30) Priorität: 18.11.2005 CH 18462005; 23.12.2005 CH 20592005; 08.06.2006 CH 9252006; 21.06.2006 CH 10042006
(43) Veröffentlichungstag der Anmeldung: 26.01.2011
(62) Teilanmeldung aus: 06804873.5
(73) Patentinhaber: Uster Technologies AG, 8610 Uster (CH)
(72) Erfinder: Meixner, Christine, 8636 Wald (CH); Peters, Gabriela, 8320 Fehraltorf (CH); Meier, Sandra, 8340 Hadlikon (CH)

(56) Entgegenhaltungen:
- WO-A-01/92875
- CH-A5- 672 931
- CH-A5- 683 350
- DE-A1- 3 237 371
- DE-A1- 3 928 417
- DE-A1- 10 348 742

## Beschreibung

### FACHGEBIET

Die Erfindung betrifft ein Verfahren zur Charakterisierung von Effektgarn, gemäss Oberbegriff des ersten Patentanspruchs. Sie kann sowohl im Textillabor (offline) als auch in der Textilproduktion (online), z. B. in Spinnerei oder Spulerei, eingesetzt werden.

### STAND DER TECHNIK

Effektgarn ist Garn, dessen Struktur oder Faserzusammensetzung von normalem Glattgarn abweicht. Es wird als bereicherndes Element in Weberei- und Strickereiprodukten eingesetzt. Üblicherweise weist Effektgarn eine Vielzahl von Dickstellen oder Dünnstellen - so genannten Effekten - auf, deren Durchmesser deutlich grösser bzw. kleiner ist als der Durchmesser der zwischen zwei Effekten liegenden Garnabschnitte - der so genannten Stege. Aber auch strukturiertes Garn mit absichtlich erzeugten Dickenvariationen, bei dem keine Stege identifiziert werden können, wird zum Effektgarn gezählt. Die zunehmende Beliebtheit von Effektgarn verlangt nach zuverlässigen und aussagekräftigen Verfahren zu dessen Charakterisierung. Insbesondere interessieren Grössen wie Stegdurchmesser, Durchmesserzunahme bei einem Effekt, Effektmasse, Effektlänge, Steglänge etc. Diese Grössen können z. B. zur Kontrolle der Qualität eines vorliegenden Effektgarns oder zur Bestimmung der Herstellungsparameter, die zum Kopieren eines gegebenen Effektgarns nötig sind, verwendet werden.

Verfahren und Vorrichtungen zur Charakterisierung von Garn sind bekannt. Sie basieren üblicherweise auf einer kapazitiven und/oder optischen Abtastung des in Längsrichtung bewegten Garns. Das kapazitive Abtastprinzip liefert ein der Garnmasse entsprechendes Signal, während das optische Abtastprinzip ein zum Garndurchmesser proportionales Signal liefert. Das Abtastsignal wird analog und/oder digital ausgewertet, und ein oder mehrere Resultate der Auswertung wird bzw. werden ausgegeben. Beispiele für derartige Verfahren und Vorrichtungen zur Charakterisierung von Garn sind in den Patentveröffentlichungen

EP-0'578'975 A 1 oder EP-0'249'741 A2 angegeben; beide beziehen sich auf das Garnprüfsystem USTER^{®} TESTER, das weltweit von der Inhaberin des vorliegenden Schutzrechtes vertrieben wird.

Die DE-39'28'417 A 1 und die CH-683'350 A5 zeigen jeweils ein Klassierfeld zur Erfassung von Garnfehlern an einem Garn. Das Klassierfeld wird durch die Fehlerlänge sowie die Fehlerdicke aufgespannt und ist in eine Vielzahl von rechteckigen Klassen unterteilt. In die einzelnen Klassen werden Zählwerte für die jeweiligen Garnfehler eingetragen.

Die WO-01/92875 A1 offenbart ein Verfahren und eine Vorrichtung zur Erkennung von Fremdstoffen in einem Garn. Es werden zwei verschiedene Parameter am Garn erfasst, insbesondere die Reflektivität und die Garnmasse. Die Messwerte können in einem zweidimensionalen Scatterplot dargestellt werden, dessen Achsen von den beiden Parametern aufgespannt werden. Dies ermöglicht die Unterscheidung verschiedener Fremdstoffe.

Gemäss der DE-32'37'371 A 1 wird das an einem Faden aufgenommene Signal eines optischen Dickstellenfängers digitalisiert und einer Spektralanalyse sowie einer Integralanalyse unterzogen. Bei der Spektralanalyse wird ein Signalspektrum berechnet, das eine Beurteilung der zyklischen Fadenungleichmässigkeiten erlaubt. Bei der Integralanalyse wird der Absolutwert der Abweichung von einem festgelegten Sollwert über eine bestimmte Garnlänge integriert; der so erhaltene Integralwert ist ein Mass für die Garnungleichmässigkeit. Ausserdem wird eine Glättung des Signals durch einen Tiefpassfilter beschrieben.

Es ist auch bekannt, Information über die Farbe, d. h. über die spektralen Reflexionseigenschaften, von Garn zu gewinnen. So wird z. B. gemäss der WO-2004/044579 eine mehrfarbige Lichtquelle zur Beleuchtung des Garns verwendet. Das vom Garn reflektierte Licht wird in mindestens zwei verschiedenen Spektralbereichen getrennt detektiert. Die mindestens zwei Detektionssignale erlauben eine Aussage über die Garnfarbe. Eine gleichzeitige optische Abtastung eines textilen Materials mit verschiedenen Wellenlängen ist auch aus der CH 674'379 oder aus der DE-198'59'274 bekannt.

Speziell mit Effektgarn befassen sich die WO-2005/071'150 A1, die WO-2005/038'105 A 1 und die WO-2005/037699 A 1. Die Lehre der Letzteren kann wie folgt zusammengefasst werden:
- Bestimmung des Stegdurchmessers: Zunächst wird der arithmetische Mittelwert des Garndurchmessers über eine grosse Garnlänge gebildet. Dieser Mittelwert wird von den Einzelwerten des Garndurchmessers subtrahiert. Als Stegdurchmesser wird der arithmetische Mittelwert aus allen negativen Werten, die benachbart zu anderen negativen Werten gemessen wurden, definiert.
- Bestimmung des Beginns, des Endes und der Länge eines Effektes: Ein Effektbeginn liegt dann vor, wenn ein Grenzdurchmesser überschritten wird, der über dem Stegdurchmesser liegt, und das Überschreiten über eine gewisse Garnlänge andauert. Ein Effektende liegt dann vor, wenn der Grenzdurchmesser unterschritten wird und das Unterschreiten über eine gewisse Garnlänge andauert. Die Effektlänge wird als Distanz zwischen Effektbeginn und Effektende definiert.
- Bestimmung des Effektdurchmessers: Innerhalb eines Effektes wird eine Mehrzahl grösster Durchmesser ermittelt. Der Effektdurchmesser wird als Mittelwert dieser grössten Durchmesser definiert.
Obwohl diese Lehre eine brauchbare Charakterisierung von Effektgarn ermöglicht, hat sie auch einige Nachteile. Als Resultat fällt eine grosse Datenmenge an, die unübersichtlich und schwer handhabbar ist. Geeignete Möglichkeiten zur übersichtlichen Darstellung der Messresultate werden nicht angegeben. Das beschriebene Verfahren liefert einen eher zu grossen Stegdurchmesser und eine eher zu kleine Effektlänge.

### DARSTELLUNG DER ERFINDUNG

Es ist eine Aufgabe der vorliegenden Erfindung, ein Verfahren zur Charakterisierung von Effektgarn anzugeben, das eine übersichtliche Darstellung der Messresultate liefert. Die anfallenden Daten sollen gegenüber der Lehre der WO-2005/037699 A1 reduziert werden. Ferner soll es die Erfindung ermöglichen, die anfallenden Daten auf geeignete Weise in bestimmte Kategorien aufzuteilen, z. B. in Daten, welche die Effekte betreffen, und Daten, welche Garnimperfektionen betreffen.

Diese und andere Aufgaben werden durch das in Anspruch 1 definierte Verfahren gelöst. Vorteilhafte Ausführungsformen sind in den abhängigen Patentansprüchen angegeben.

Gemäss dem erfindungsgemässen Verfahren zur Charakterisierung von Effektgarn, das vorzugsweise eine Abfolge von Effekten und Stegen aufweist, wird mindestens eine Eigenschaft des Effektgarns entlang der Längsrichtung des Effektgarns abgetastet. Es werden Werte der Abtastung ausgewertet, Resultate der Auswertung werden ausgegeben. Mindestens ein Resultat der Auswertung wird in Form einer grafischen Darstellung ausgegeben. Die grafische Darstellung ist eine Fläche, die in einem dreidimensionalen Raum liegt, dessen erste Dimension eine Effektlänge, dessen zweite Dimension eine Massenzunahme oder eine Durchmesserzunahme von Effekten und dessen dritte Dimension eine beobachtete Häufigkeit der entsprechenden Effektcharakteristika angibt. Alternativ ist die grafische Darstellung eine Projektion einer solchen Fläche in die durch eine Effektlängenachse und eine Massenzunahmen- oder Durchmesserzunahmenachse aufgespannte Ebene. Eine derartige grafische Darstellung reduziert die anfallende Datenmenge. Sie ermöglicht ein rasches visuelles Erfassen der Resultate. Dadurch wird es auch möglich, die Daten in verschiedene Kategorien aufzuteilen. In einer bevorzugten Ausführungsform ist die mindestens eine abgetastete Eigenschaft eine Masse und/oder ein Durchmesser des Effektgarns.

### AUFZÄHLUNG DER ZEICHNUNGEN

Nachfolgend wird die Erfindung anhand der Zeichnungen detailliert erläutert.
- Figur 1: zeigt schematisch eine Vorrichtung zur Durchführung des erfindungsgemässen Verfahrens.
- Figur 2: zeigt ein Beispiel einer Reihe von Messwerten an einem Effektgarn, nämlich Gammasse versus Längskoordinate.
- Figur 3: zeigt eine Häufigkeitsverteilung der gemessenen, in Figur 2 aufgetragenen Garnmasse.
- Figur 4: zeigt einen vergrösserten Ausschnitt aus Figur 2.
- Figuren 5-12: zeigen mögliche Darstellungsarten für die Ausgabe der Garnparameter.

### AUSFÜHRUNG DER ERFINDUNG

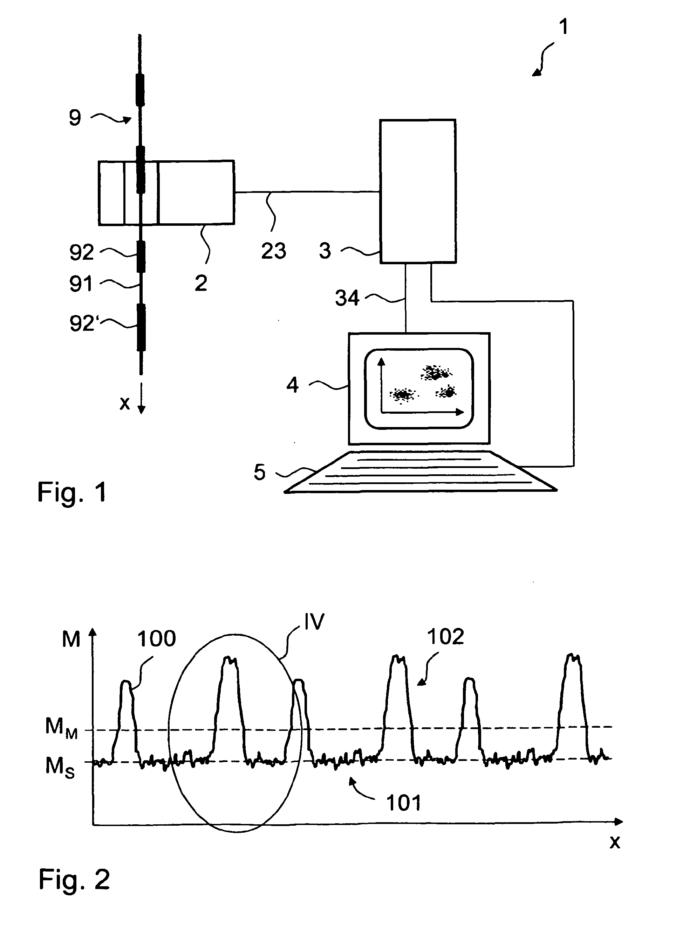

In **Figur 1** ist schematisch eine Vorrichtung 1 zur Durchführung des erfindungsgemässen Verfahrens dargestellt. Sie beinhaltet eine Abtasteinheit 2 zum Abtasten eines in Längsrichtung -x bewegten Effektgarns 9 mit Stegen 91 und Effekten 92, 92'. Unter dem Begriff "Abtasten" wird hier die sequenzielle Aufnahme einer Vielzahl von Messwerten an verschiedenen, vorzugsweise äquidistanten Stellen des Effektgarns 9 verstanden. Derartige Abtasteinheiten 2 sind an sich bekannt und brauchen hier nicht näher erläutert zu werden. Die Abtasteinheit 2 kann einen kapazitiven, optischen oder anderen Sensor beinhalten; es können auch mehrere gleiche oder verschiedene Sensoren innerhalb der Abtasteinheit 2 angeordnet sein. Die Abtasteinheit 2 kann mit Auswertemitteln für eine Vorauswertung der Messdaten ausgestattet sein. Sie gibt auf einer ersten Datenleitung 23 ein vorzugsweise elektrisches Ausgangssignal aus, das ein Mass ist für die Masse, die Dicke oder andere Eigenschaften des Effektgarns 9.

Die erste Datenleitung 23 mündet in eine Auswerteeinheit 3, die zum Auswerten des Ausgangssignals der Abtasteinheit 2 geeignet ist. Zu diesem Zweck beinhaltet sie geeignete analoge und/oder digitale Auswertemittel, z. B. einen Mikroprozessor. Sie kann auch weitere Mittel wie Speichermittel zum Speichern von Daten beinhalten. Die Auswerteeinheit 3 ist vorzugsweise ein Computer.

Ferner beinhaltet die Vorrichtung 1 eine Ausgabeeinheit 4 zum Ausgeben von Messdaten und/oder Resultaten der Auswertung. Die Ausgabeeinheit 4 ist mittels einer zweiten Datenleitung 34 mit der Auswerteeinheit 3 verbunden. Sie kann z. B. als Bildschirm und/oder Drucker ausgebildet sein. Vorzugsweise beinhaltet die Vorrichtung 1 auch eine Eingabeeinheit 5 zum Eingeben von Daten durch einen Benutzer. Die Eingabeeinheit 5 kann z. B. eine Tastatur, eine Maus und/oder einen Touch-Screen beinhalten.

**Figur 2** zeigt ein mögliches Ausgangssignal 100 der Abtasteinheit 2. Dabei ist bspw. eine Grösse, die ein Mass für die Masse M pro Längeneinheit des Effektgarns 9 (siehe Fig. 1) ist, gegenüber der Längskoordinate x des Effektgarns 9 aufgetragen; ein solches Massensignal M wird typischerweise von einem kapazitiven Garnsensor geliefert. Ähnlich würde eine Darstellung der Dicke des Effektgarns 9 gegenüber der Längskoordinate x aussehen, wobei die Massstäbe auf der Abszisse und der Ordinate sowie die Auflösung verschieden sein könnten; ein Dickensignal wird typischerweise von einem optischen Garnsensor geliefert. Die Kurve M(x) ist nicht notwendigerweise kontinuierlich, sondern kann aus einzelnen (in Fig. 2 nicht dargestellten) Abtastpunkten zusammengesetzt sein, die auf dem Effektgarn 9 typischerweise um einige Millimeter voneinander entfernt sind; die Entfernung hängt von der Abtastrate der Abtasteinheit 2 und von der Geschwindigkeit des Effektgarns 9 ab. Das Signal M(x) hat einen Grundpegel 101, welcher der Stegmasse M_{S} bzw. dem Stegdurchmesser entspricht. Aus dem Grundpegel 101 ragen deutlich den Effekten entsprechende Spitzen 102 heraus. Ein Mittelwert M_{M} aller Messwerte M(x) über eine grosse Garnlänge liegt wegen der Spitzen 102 deutlich über dem Grundpegel 101 und ist deshalb als Mass für die Stegmasse M_{S} ungeeignet. An dieser Stelle sei bemerkt, dass Figur 2 und die nachfolgende Diskussion nur ein mögliches, nicht einschränkendes Beispiel darstellt. Bei anderen Arten von Effektgarn ist es gar nicht mehr möglich, Stege zu identifizieren.

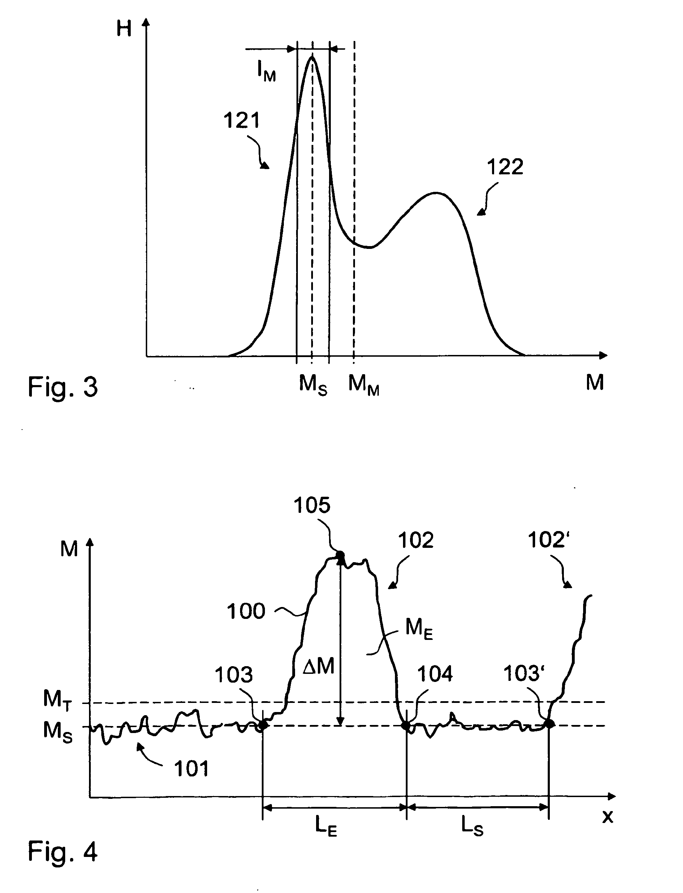

Die Stegmasse M_{S} kann vorzugsweise wie folgt ermittelt werden. Es wird eine Häufigkeitsverteilung H(M) der gemessenen und in Figur 2 dargestellten Massen M ermittelt. Eine derartige Häufigkeitsverteilung H(M) ist schematisch in **Figur 3** gezeigt. Die Häufigkeitsverteilung H(M) muss für eine grosse Garnlänge ermittelt werden, worunter hier eine Garnlänge verstanden wird, die viele, bspw. mindestens 10 und vorzugsweise mindestens 100, Effekte beinhaltet. Die Häufigkeitsverteilung H(M) weist bei einem Effektgarn 9 mindestens zwei lokale Maxima 121, 122 auf: ein erstes lokales Maximum 121 für die Stege 91 und mindestens ein zweites lokales Maximum 122 für die Effekte 92, 92', ... Definitionsgemäss hat unter allen lokalen Maxima 121, 122 das zu den Stegen 91 gehörige lokale Maximum 121 die kleinste Masse M. Als Stegmasse M_{S} wird deshalb die kleinste Masse M definiert, bei welcher in der Häufigkeitsverteilung H(M) ein lokales Maximum 121 auftritt. Aus der Stegmasse M_{S} kann die Garnnummer des Garnkörpers bzw. der Stege 91 berechnet werden. Analog kann vorgegangen werden, um einen Stegdurchmesser zu ermitteln.

Gemäss einer alternativen Ausführungsform zur Ermittlung der Stegmasse wird ein Massenintervall I_{M} (siehe Figur 3) definiert, welches diejenige kleinste Masse beinhaltet, bei welcher in der Häufigkeitsverteilung (H(M)) ein lokales Maximum 121 auftritt,. Das Massenintervall IM kann, muss aber nicht bezüglich der "kleinsten" Masse symmetrisch sein, d. h. die "kleinste" Masse kann, muss aber nicht in der Mitte des Massenintervalls IM liegen. Die obere Grenze des Massenintervalls IM wird vorzugsweise unterhalb des globalen Massenmittelwertes M_{M} gewählt. Breite und Lage des Massenintervalls I_{M} können fest vorgegeben sein - bspw. ±5 % der "kleinsten" Masse -, automatisch von der Auswerteeinheit 3 berechnet oder durch einen Benutzer eingegeben werden. Anschliessend wird ein Mittelwert über alle in diesem Massenintervall I_{M} gemessenen Massen M gebildet. Die Stegmasse wird als dieser Mittelwert definiert.

**Figur 4** zeigt in detaillierterer Ansicht einen Ausschnitt aus der Messkurve M(x) von Fig. 2. Anhand von Figur 4 wird nachfolgend erläutert, wie weitere Effektgarnparameter wie Effektlänge L_{E}, Steglänge L_{S}, Massenzunahme ΔM und Effektgesamtmasse M_{E} ermittelt werden.

Zur Feststellung eines Beginns 103 und eines Endes 104 eines Effektes 92 wird vorgängig ein Schwellenwert M_{T} festgelegt, der grösser ist als die Stegmasse M_{S}. Der Schwellenwert M_{T} kann fest vorgegeben sein - bspw. 110 % der Stegmasse M_{S} -, automatisch von der Auswerteeinheit 3 berechnet oder durch einen Benutzer eingegeben werden. Der Beginn 103 eines Effektes 92 liegt dann vor, wenn ausgehend vom Grundpegel 101 der Schwellenwert M_{T} überschritten wird. Um Artefakte durch Ausreisser auszuschliessen, kann zusätzlich kontrolliert werden, ob das Überschreiten über eine vorbestimmte Garnlänge andauert, d. h. ob einige weitere Messpunkte, die unmittelbar auf den den Schwellenwert M_{T} überschreitenden Messpunkt folgen, ebenfalls über dem Schwellenwert M_{T} liegen. Zur exakteren Ermittlung des Beginns 103 des Effektes 92 geht man auf der Messkurve M(x) so weit zurück, bis erstmals ein Messwert kleiner oder gleich der Stegmasse M_{S} - oder einem anderen, vordefinierten oder berechneten Wert - ist. Dieser Messwert wird als Beginn 103 des Effektes 92 definiert. In einem analogen Verfahren wird, mutatis mutandis, das Ende 104 des Effektes 92 ermittelt: Unterschreiten des Schwellenwertes M_{T} ausgehend von der Signalspitze 102, auf der Messkurve M(x) so weit vorwärts, bis erstmals ein Messwert kleiner oder gleich der Stegmasse M_{S} ist. Bei Bedarf können zur Feststellung des Effektbeginns 103 und des Effektendes 104 unterschiedliche Schwellenwerte verwendet werden.

Die Effektlänge L_{E} wird als Entfernung zwischen dem Beginn 103 und dem Ende 104 des Effektes 92 definiert. Die Steglänge L_{S} wird als Entfernung zwischen dem Ende 104 eines Effektes und dem Beginn 103' eines nachfolgenden Effektes 92', zu dem eine nachfolgende Signalspitze 102' gehört, definiert. Die Entfernung zwischen zwei benachbarten Effekten 92, 92' wird als die Summe L_{E} + L_{S} von Effektlänge und Steglänge definiert. Typische Effektlängen L_{E} und Steglängen L_{S} liegen im Bereich zwischen 2 cm und einigen Metern.

Die Massenzunahme ΔM, die ungefähr einer Durchmesserzunahme des Effektgarns 9 entspricht, wird im einfachsten Fall als Differenz zwischen einem lokalen Maximum 105 der entsprechenden Signalspitze 102 und der Stegmasse M_{S} definiert. Zur Ermittlung der Massenzunahme ΔM sind auch verfeinerte Verfahren möglich, die Schwankungen des Abtastsignals M(x) entlang der Effektlänge Rechnung tragen. So könnte zum Beispiel - analog zur oben beschriebenen Ermittlung der Stegmasse M_{S} - der häufigste Wert innerhalb der entsprechenden Effektlänge ausgewählt werden. Alternativ kommt eine Mittelwertbildung von Werten auf dem Effektkamm in Frage. Die Massenzunahme ΔM wird vorzugsweise als Vielfaches der Stegmasse M_{S} angegeben, z. B. in Prozentwerten, wobei die Stegmasse M_{S} vorzugsweise als 100 % definiert ist. Typische Massenzunahmen ΔM liegen im Bereich zwischen 20 % und 500 %.

Ein weiterer Parameter zur Charakterisierung eines Effektes 92 ist die so genannte Effektgesamtmasse M_{E}. Diese ist im Wesentlichen die Differenz zwischen (i) dem Integral der Messkurve M(x) über die Effektlänge L_{E} und (ii) der Masse M_{S}·L_{E} des Garnkörpers auf dieser Effektlänge L_{E}. Die Effektgesamtmasse M_{E} kann rechnerisch von der Auswerteeinheit 3 ermittelt werden. Aus der Effektgesamtmasse M_{E} kann die Garnnummer des Effektes 92 berechnet werden, wobei die Berechnung eine Division der Effektgesamtmasse M_{E} durch die Effektlänge L_{E} beinhalten kann.

Auch die Form eines Effektes 92 kann ermittelt und ausgegeben werden. Dabei kann auf einen Vergleich mit einfachen geometrischen Formen wie Säule, Dreieck, Treppe, Trapez oder Glocke zurückgegriffen werden; vgl. Fig. 18. Die entsprechende Form kann z. B. auf der Ausgabeeinheit 4 ausgegeben werden.

Es interessieren nicht nur "lokale" Parameter der einzelnen Effekte, sondern auch "globale" Parameter eines ganzen Garnabschnitts, eines Garns oder einer Gruppe von mehreren Garnen. Ein solcher globaler Effektgarnparameter ist die mittlere Garnnummer (mittlere Garnmasse), die sich bspw. durch Mittelwertbildung über alle Messwerte berechnen lässt. Auch die Garnnummer aller Stege 91 kann interessieren. Ein weiterer globaler Effektgarnparameter ist die mittlere räumliche Frequenz der Effekte, d. h. die mittlere Anzahl Effekte pro Längeneinheit.

Die oben genannten und möglicherweise weitere Effektgarnparameter werden vorzugsweise dynamisch, während der Laufzeit der Abtastung, ermittelt. Die Effektparameter werden zwecks Ausgabe gespeichert. Es ist von Vorteil, zusätzlich auch eine durchgehende, dem jeweiligen Effekt zugeordnete Laufnummer zu speichern, um Information nicht nur über die einzelnen Effekte, sondern auch über ihre Reihenfolge zur Verfügung stellen zu können.

Es kann vorteilhaft sein, die Abtasteinheit 2 (Fig. 1) sowohl mit einem kapazitiven als auch mit einem optischen Garnsensor auszustatten, die gleichzeitig ein und dasselbe Garn ausmessen können. Die Ausgangssignale des kapazitiven und des optischen Sensors können zwecks einer besseren oder genaueren Auswertung auf geeignete Weise miteinander verknüpft werden. Die kapazitive Messung hat den Vorteil, dass sie ein Signal mit einem guten Signal/Rauschverhältnis liefert. Das Signal ist aber proportional zur Masse pro Längeneinheit und entspricht somit nicht dem visuellen Eindruck vom Garn; dies wirkt sich gerade bei Effektgarn, das im Bereich eines Effektes 92 oft eine andere Garndichte aufweist als im Bereich eines Stegs 91, nachteilig aus. Die optische Messung hat den Vorteil, den visuellen Eindruck vom Garn besser wiederzugeben, weil sie im Wesentlichen den sichtbaren Garndurchmesser misst; deshalb eignet sie sich besser für Gewebesimulationen. Dafür weist das optische Messsignal ein höheres Rauschen auf als das kapazitive Messsignal. Durch geeignete Verknüpfung der beiden Ausgangssignale ist es möglich, von den Vorteilen beider Messarten zu profitieren und ihre jeweiligen Nachteile zu eliminieren oder zumindest abzuschwächen.

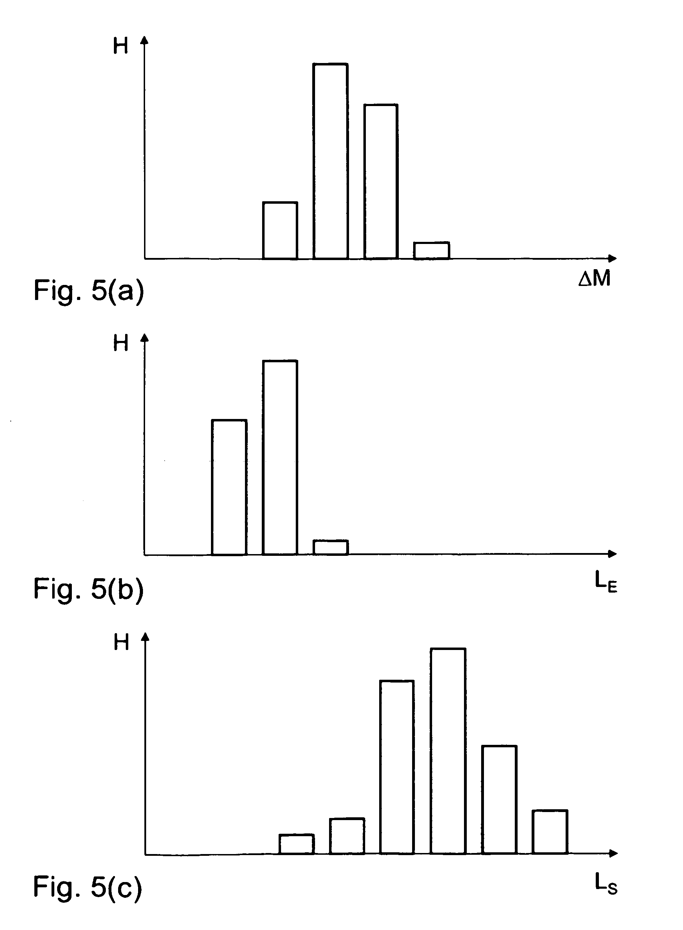

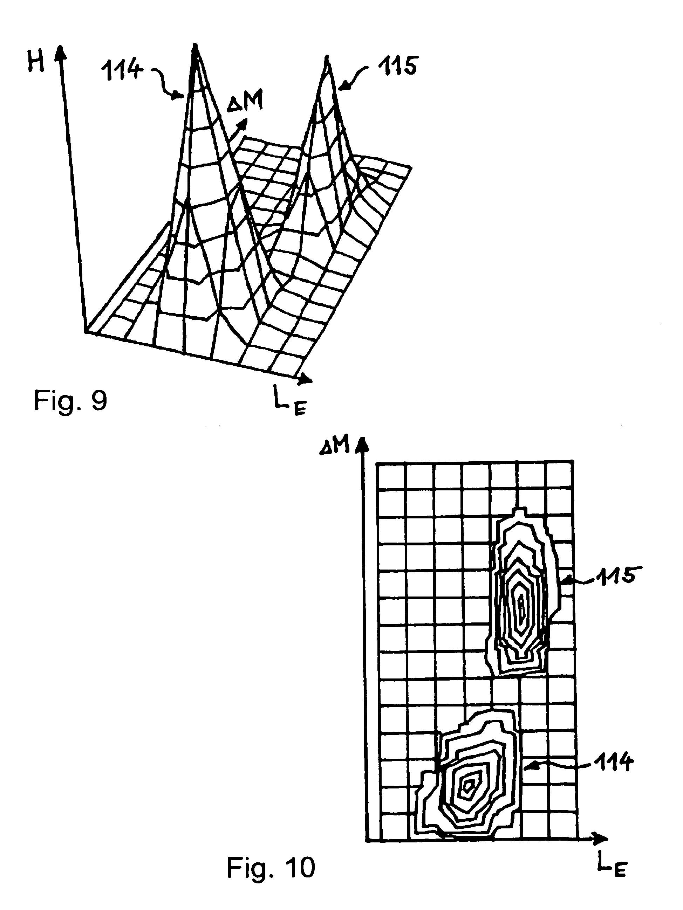

Resultate der Auswertung können einerseits Grössen sein wie z. B. Minima, Maxima, arithmetische Mittel und/oder Standardabweichungen der oben definierten Effektgarnparameter. Die Anzahl Effekte 92 pro Garnlänge kann eine weitere interessierende Grösse sein. Diese Grössen können als alphanumerische Zeichen ausgegeben werden. Andererseits können die Resultate der Auswertung in geeigneter Weise auf der Ausgabeeinheit 4 grafisch dargestellt und ausgegeben werden. Bevorzugte Darstellungsarten sind in den Figuren 5-12 gezeigt, wobei sich nur Figuren 9 und 10 auf die in Anspruch 1 definierte Erfindung beziehen.

Eine mögliche Darstellungsart ist ein Histogramm, d. h. die grafische Darstellung der Klassenhäufigkeiten der klassierten Garnparameter. Drei Beispiele in Form von Säulendiagrammen sind in **Figur 5** angegeben. Die Ordinate ist jeweils die Häufigkeit H. Als Abszisse wurde in **Figur 5(a)** die Massenzunahme ΔM, in **Figur 5(b)** die Effektlänge L_{E} und in **Figur 5(c)** die Steglänge L_{S} verwendet. Die Abszissen können eine lineare, logarithmische oder andere Einteilung aufweisen. Einteilung und/oder Skalierung der Achsen können automatisch berechnet oder mittels Eingabe durch eine Bedienungsperson gewählt werden. Dasselbe gilt für die Wahl der Klassierung, d. h. für die Breite der Klassen. Es müssen nicht notwendigerweise alle Klassen dieselbe Breite aufweisen.

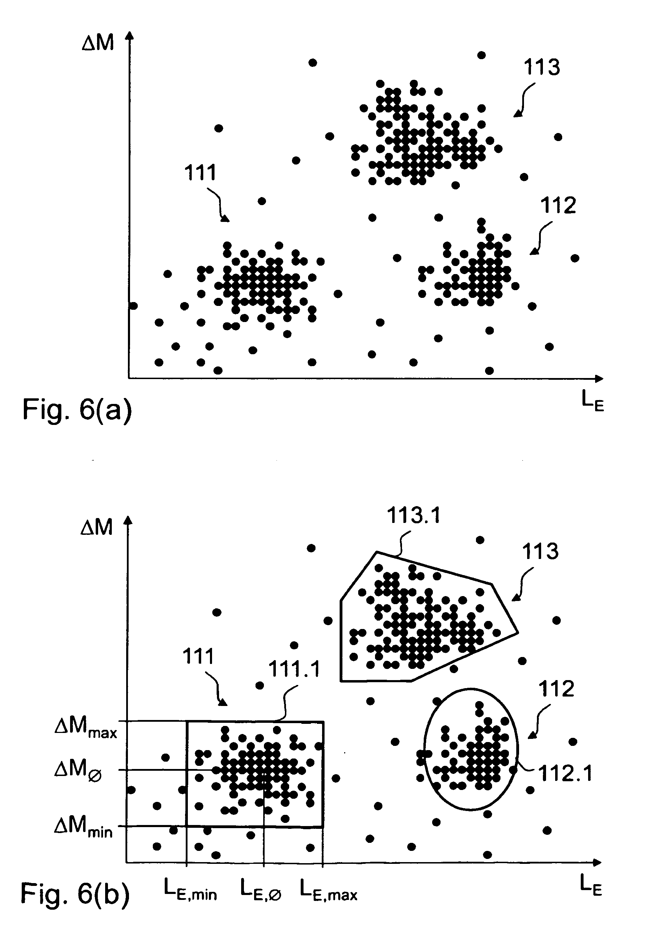

Die Darstellungsart von **Figur 6** ist ein so genannter Scatterplot (Punktwolke). Darin ist für alle Effekte 92 eines Effektgarns 9 oder eines Garnabschnitts die Massenzunahme ΔM gegenüber der Effektlänge L_{E} aufgetragen. Jeder Effekt 92 wird als Punkt an der richtigen Stelle eingetragen. Diese Darstellung erleichtert die Einteilung verschiedener Effekte 92 in verschiedene Klassen. So ist im Beispiel von **Figur 6(a)** sofort ersichtlich, dass das untersuchte Effektgarn 9 drei Klassen 111-113 oder Populationen von Effekten 92 aufweist:
- eine erste Klasse 111 mit kurzen, niedrigen Effekten,
- eine zweite Klasse 112 mit langen, niedrigen Effekten, und
- eine dritte Klasse 113 mit langen, hohen Effekten.

Anhand des Scatterplots von Fig. 6(a) kann eine Ausreisseranalyse vorgenommen werden. Zu diesem Zweck kann ein Werkzeug vorgesehen sein, um Effektpopulationen 111-113 zu definieren und voneinander sowie von Ausreissern abzugrenzen. Ein solches Werkzeug kann es z. B. ermöglichen, auf einem Bildschirm mit einer Maus Teilflächen 111.1-113.1 des Scatterplots zu definieren, wie in **Figur 6(b)** dargestellt. Jeder Teilfläche 111.1-113.1 wird eine Effektpopulation 111-113 zugewiesen. Die Teilflächen können z. B. als Rechteck 111.1, als Kreis 112.1 oder als Polygonzug 113.1 geformt sein. Ausserhalb der Teilflächen 111.1-113.1 liegende Punkte gelten als Ausreisser und können bei weiteren Auswertungen und/oder Darstellungen als solche gekennzeichnet werden oder unberücksichtigt bleiben. Wird keine Teilfläche definiert, so gelten alle Punkte des Scatterplots als Effekte und werden als solche behandelt.

Andere Scatterplots sind möglich, bspw. Effektgesamtmasse M_{E} versus Effektlänge L_{E}, Effektgesamtmasse M_{E} versus Massenzunahme ΔM etc. Der Scatterplot kann farbig ausgegeben werden, wobei verschiedene Farben verschiedene Messungen, verschiedene Punktdichten und/oder Populationen bzw. Ausreisser (vgl. Fig. 10) bedeuten können. Analog ist eine dreidimensionale Darstellung möglich, in der zwei Koordinaten denjenigen von Fig. 6 entsprechen und die dritte Koordinate der Punktdichte entspricht; vgl. Fig. 9.

Der Scatterplot kann für eine einzelne Effektgarnprobe oder für mehrere Effektgarnproben dargestellt werden. Im letzteren Fall können verschiedene Farben für die verschiedenen Effektgarnproben verwendet werden, um mögliche Unterschiede zwischen den Proben aufzuzeigen. Bei mehreren Effektgarnproben kann zusätzlich zu den Resultaten der einzelnen Effektgarnproben das Resultat der Gesamtheit aller Effektgarnproben angezeigt werden, vorzugsweise in einer eigens zugeordneten Farbe.

Auf dem Scatterplot können nebst den Istwerten auch Sollwerte oder Sollbereiche für eine oder mehrere Klassen von Effekten dargestellt werden. Soll- und Istwerte können auch in anderen Darstellungsarten oder rein numerisch verglichen werden. Derartige Soll-Ist-Vergleiche ermöglichen z. B. eine Kontrolle über die Güte einer Kopie (Istwert) eines vorgegebenen Effektgarns (Sollwert).

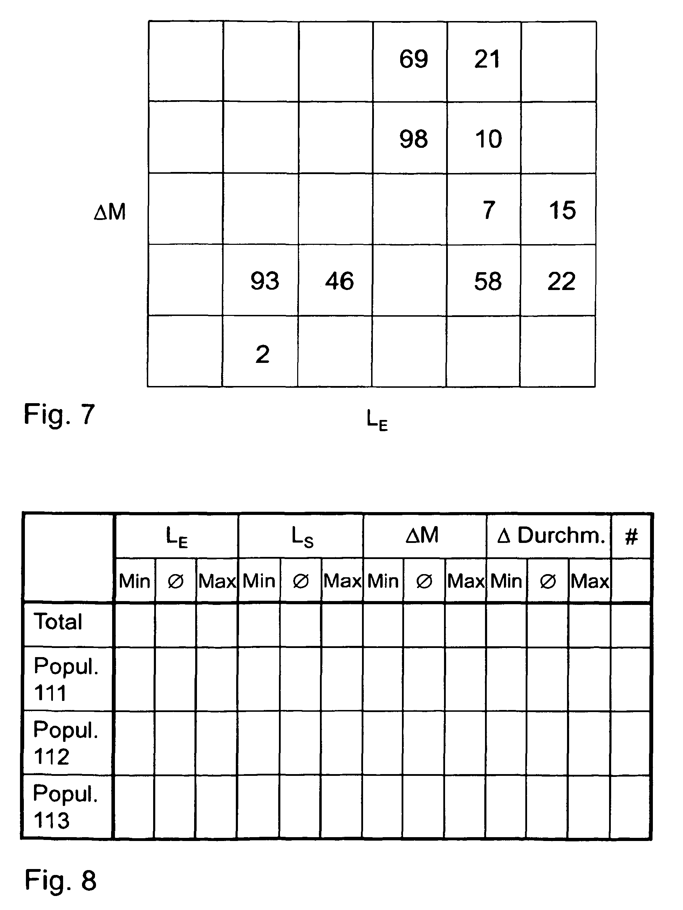

Statt eines Scatterplots können die Resultate in Form einer Tabelle oder Klassiermatrix - wie in Figur 7 dargestellt - ausgegeben werden. Die Tabellenachsen entsprechen den Achsen des Scatterplots von Fig. 6. In die Tabellenfelder werden die Anzahlen von entsprechenden Effekten 92 eingetragen. Alternativ zur absoluten Anzahl von Effekten 92 kann auch deren relativer Anteil, z. B. in Prozent oder Promille, angegeben werden. Jedes Tabellenfeld stellt somit eine Klasse von Effekten 92 dar, analog zu den anlässlich von Fig. 6 beschriebenen Klassen 111-113. Die gewählte Grösse der Tabellenfelder richtet sich nach der angestrebten Klassierung. Bei der Wahl der Grösse der Tabellenfelder sollte ferner berücksichtigt werden, dass die Auflösung genügend fein ist, aber die Tabelle noch übersichtlich bleibt. Im Beispiel von Figur 7 wurden relativ kleine Tabellenfelder gewählt, die eine feinere Klassierung erlauben als in die drei Klassen 111-113, die anlässlich von Figur 6 identifiziert wurden. Zwecks besserer Visualisierung können die Tabellenfelder mit Farben, Mustern oder Graustufen gefüllt werden, die wiederum verschiedenen Anzahlen von Effekten 92 zugeordnet sind.

Sowohl beim Scatterplot (Fig. 6) als auch bei der Klassiermatrix (Fig. 7) können die Achsen, unabhängig voneinander, eine lineare, logarithmische oder andere Einteilung aufweisen. Einteilung und/oder Skalierung der Achsen können automatisch berechnet oder mittels Eingabe durch eine Bedienungsperson gewählt werden. Dasselbe gilt für Breite und Höhe der Tabellenfelder in der Klassiermatrix von Figur 7, d. h. für die Wahl der Klassierung.

**Figur 8** zeigt eine bevorzugte Ausgabemöglichkeit für die ermittelten Effektgarnparameter. Es handelt sich um eine Tabelle, die in fünf Hauptkolonnen für die fünf Parameter Effektlänge L_{E}, Steglänge L_{S}, Massenzunahme ΔM, Durchmesserzunahme und Anzahl # der Effekte unterteilt ist. Die ersten vier Hauptkolonnen sind ihrerseits in jeweils drei Unterkolonnen für das Minimum Min, den Mittelwert Ø und das Maximum Max des entsprechenden Parameters unterteilt. Die Zeilen der Tabelle können die Werte für das gesamte Garn oder den gesamten untersuchten Garnabschnitt, sowie für die einzelnen Effektpopulationen 111-113 enthalten. Die Minima L_{E,min}, ΔMₘᵢₙ, die Mittelwerte L_{E,Ø}, ΔM_{Ø} und die Maxima L_{E,max}, ΔMₘₐₓ sind für die Population 111 in Fig. 6(b) angedeutet. Selbstverständlich kann die Tabelle auch anders gestaltet sein. Jedenfalls führt eine solche tabellarische Darstellung der ermittelten Effektgarnparameter zu einer Datenreduktion. Anhand der Tabelle lassen sich Besonderheiten des betreffenden Effektgarns schnell erfassen, und verschiedene Effektgame lassen sich leicht vergleichen.

Eine weitere Darstellungsart für Resultate der Auswertung von Effektgarnparametern zeigt **Figur 9** Es handelt sich um eine Fläche in drei Dimensionen (3D). Zwei der drei Dimensionen, die beiden horizontalen Achsen, entsprechen der Effektlänge L_{E} bzw. der Massenzunahme ΔM, wie im Scatterplot von Fig. 6. Die dritte, vertikale Dimension gibt die entsprechende Häufigkeit H der gemessenen Werte an, d. h. die Punktdichte im Scatterplot von Fig. 6 oder die Anzahlen von Fig. 7. Die so entstandene 3D-Fläche vermittelt den Eindruck eines Gebirges, das eine rasche und einprägsame visuelle Erfassung der Eigenarten des jeweiligen Effektgarns 9 erlaubt. Besonders ein synoptischer Vergleich zweier solcher "Gebirge" zeigt sehr schnell, ob die betreffenden Effektgarne ähnliche oder unterschiedliche Charakteristika aufweisen, und im letzteren Fall, wo die Hauptunterschiede liegen. Zu bemerken ist, dass sich das Beispiel von Fig. 9 auf ein anderes Effektgarn bezieht als das Beispiel von Fig. 6. Während das Effektgarn von Fig. 6 drei Klassen 111-113 von Effekten 92 aufweist, hat das Effektgarn von Fig. 9 nur deren zwei 114, 115.

Die dreidimensionale Darstellungsart von Fig. 9 lässt sich auch auf zwei Dimensionen reduzieren. **Figur 10** zeigt ein solches Diagramm, das durch eine Projektion des "Gebirges" von Fig. 9 in die durch die beiden horizontalen Achsen L_{E}, M aufgespannte Ebene entsteht. So ergibt sich eine "Landkarte", auf der die "Berge" 114, 115 von Fig. 9 mittels "Höhenlinien", d. h. Linien gleicher Häufigkeit H, dargestellt sind. Statt "Höhenlinien" können Farben oder Schraffuren zur Sichtbarmachung der verschiedenen Häufigkeiten H verwendet werden.

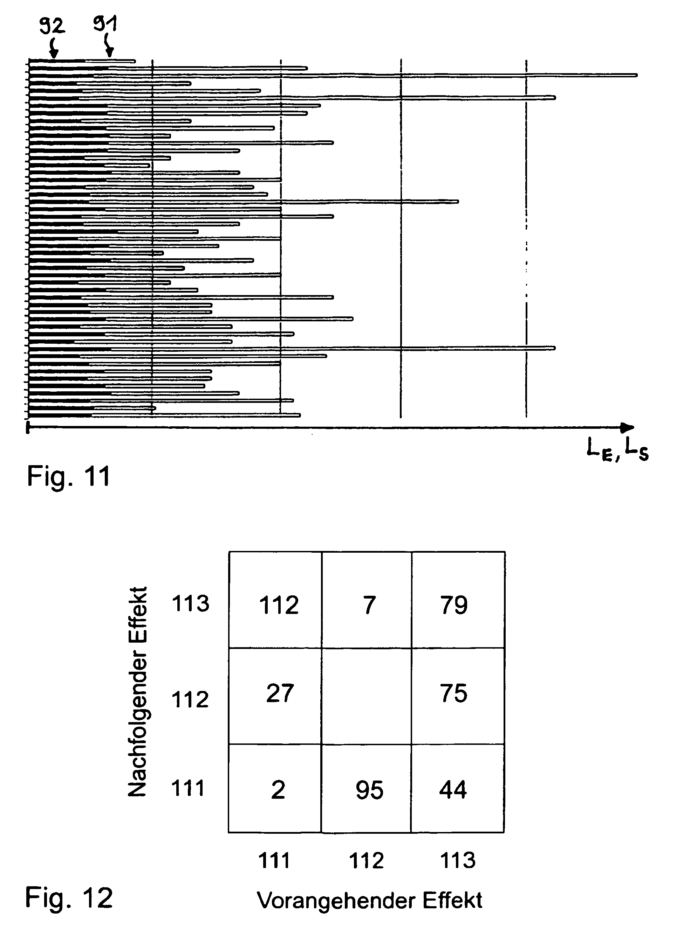

Die Darstellungsarten der Figuren 5-10 lassen Information über die Abfolge der einzelnen Effekte unberücksichtigt. Diese Information ist in der Messreihe, wie sie in Figur 2 dargestellt ist, vollständig vorhanden. Es ist möglich, daraus die entsprechenden Garnparameter wie Massenzunahme, Effektlänge und Steglänge als Messgrössen (Zahlenwert x Einheit) zu ermitteln und diese Messgrössen in der Reihenfolge ihres Auftretens nacheinander aufzulisten. Eine solche alphanumerische Auflistung der reduzierten Messdaten kann für bestimmte Fälle nützlich sein, ist jedoch wenig übersichtlich. In der Darstellungsart von **Figur 11** ist die Information über die Abfolge der einzelnen Effekte vollständig erhalten, wobei die Grafik gegenüber einer alphanumerischen Wertetabelle den Vorteil einer besseren Übersichtlichkeit und visuellen Erfassbarkeit bietet. Für jeden Effekt 92 und einen benachbarten Steg 91 ist jeweils ein horizontaler Balken eingezeichnet. Der Balken setzt sich aus zwei Teilen zusammen. Die Länge eines ersten, linken Teils gibt die jeweilige Effektlänge L_{E} an, die Länge eines zweiten, rechten Teils die jeweilige Steglänge L_{S}. Der nächste, darunter liegende Balken charakterisiert den nachfolgenden Effekt, usw. Selbstverständlich könnten die horizontalen Balken von Fig. 11 durch vertikale Säulen ersetzt werden. Statt Längen L_{E}, L_{S} können andere Messgrössen als Balken aufgetragen werden, z. B. eine Effektmasse und die dazugehörige Stegmasse, was besonders bei strukturiertem Garn mit unterschiedlichen Stegmassen vorteilhaft sein kann. Auch ist es möglich, dass ein Balken oder eine Säule mehr als zwei Effektparameter angibt, z. B. bei mehrstufigen Effekten die erste Effektlänge L_{E.1}, die zweite Effektlänge L_{E.2} und die dazugehörige Steglänge L_{S}.

**Figur 12** zeigt eine Darstellungsart, welche zumindest einen Teil der Information über die Abfolge der einzelnen Effekte übersichtlich wiedergibt. Es handelt sich um eine Klassiermatrix, die eine Klassierung der Effekte voraussetzt, wie sie etwa in Figur 6 mit den Klassen 111-113 vorgenommen wurde. Es wird jeweils ein Paar von zwei benachbarten Effekten 92, 92' betrachtet, von denen ein erster Effekt 92 "vorangehender Effekt" und ein zweiter Effekt 92' "nachfolgender Effekt" genannt wird. Für jedes Paar 92, 92' erfolgt ein entsprechender Eintrag in die Klassiermatrix von Figur 12, deren horizontale Achse die vorangehenden Effekte 92 und deren vertikale Achse die nachfolgenden Effekte 92' angibt. Aus dem fiktiven Beispiel von Figur 12 kann man herauslesen, dass praktisch nie zwei Effekte der ersten Klasse 111 (vgl. Fig. 6) und nie zwei Effekte der zweiten Klasse 112 aufeinander folgen, dass hingegen oft ein Effekt der ersten Klasse 111 auf einen Effekt der zweiten Klasse 112 folgt und sehr oft ein Effekt der dritten Klasse 113 auf einen Effekt der ersten Klasse 111 folgt.

Es kann im erfindungsgemässen Verfahren von Vorteil sein, die Messwerte, wie sie etwa in Figur 2 oder 4 dargestellt sind, nach bestimmten Filterkriterien zu filtern. Solche Filterkriterien können bspw. folgende sein:
- Garnimperfektionen wie Nissen, Dick- und/oder Dünnstellen. So ist es etwa aus der CH-678'173 A5 oder der US-5,537,811 A bekannt, zum Einstellen der Reinigungsgrenze eines elektronischen Garnreinigers mögliche Garnfehler in einer Tabelle nach Art eines Koordinatensystems anzuordnen. Die Abszisse des Koordinatensystems stellt die Fehlermasse und die Ordinate die Fehlerlänge dar. In dieses Koordinatensystem werden eine obere und eine untere Reinigungsgrenze gelegt. Nissen und Dickstellen oberhalb der oberen Reinigungsgrenze und Dünnstellen unterhalb der unteren Reinigungsgrenze werden aus dem Garn automatisch entfernt. In analoger Weise kann auch beim erfindungsgemässen Verfahren vorgegangen werden, indem mindestens eine "Reinigungsgrenze" definiert wird. Dick- bzw. Dünnstellen innerhalb dieser Grenze werden herausgefiltert und nur Effekte ausserhalb dieser Grenze werden weiter ausgewertet und/oder grafisch dargestellt.
- Effektcharakteristiken. So können z. B. Effekte herausgefiltert werden, die eine bestimmte Effektlänge L_{E} unter- oder überschreiten, die eine bestimmte Effektgesamtmasse M_{E} unter- oder überschreiten, die eine bestimmte Effektform aufweisen oder nicht aufweisen, etc. Nur die verbleibenden, nicht herausgefilterten Effekte werden weiter ausgewertet und/oder grafisch dargestellt. Es ist auch möglich, mehrere verschiedene Filter vorzusehen, so dass bspw. mit einem ersten Filter nur kurze Effekte und mit einem zweiten Filter nur lange Effekte ausgewertet und/oder grafisch dargestellt werden können.

Vorzugsweise erlaubt das erfindungsgemässe Verfahren eine interaktive Eingabe bestimmter Parameter durch eine Bedienungsperson. Solche einzugebende Parameter können etwa Filterparameter für die oben diskutierten Filter sein. Auch Grundlagen für die Auswertung können als Parameter eingegeben werden, so z. B. eine vorgegebene Stegmasse M_{S}.

Es kann vorteilhaft sein, im erfindungsgemässen Verfahren eine Schnittstelle zur Ausgabe von Daten, die im Verfahren gewonnen wurden, vorzusehen. Solche Daten können z. B. die oben diskutierten Effektgarnparameter sein, die an eine Simulationssoftware übergeben werden. Die Simulationssoftware kann daraus eine Simulation des untersuchten Garns und eines aus dem Garn gewebten oder gestrickten Flächengebildes sein. Eine solche auf Auswertedaten basierende Simulation ist, verglichen mit einer auf Messdaten basierenden Simulation, verhältnismässig schnell und einfach.

Selbstverständlich ist die vorliegende Erfindung nicht auf die oben diskutierten Ausführungsformen beschränkt. Bei Kenntnis der Erfindung wird der Fachmann weitere Varianten herleiten können, die auch zum Gegenstand der vorliegenden Erfindung gehören, soweit sie unter den Schutzumfang der Ansprüche fallen. Einzelne oben beschriebene Merkmale des erfindungsgemässen Verfahrens, insbesondere die Auswertealgorithmen für die einzelnen Effektgarnparameter, können auch losgelöst von der erfindungsgemässen grafischen Darstellung Anwendung finden. Obwohl sich die vorliegende Beschreibung auf das Beispiel der kapazitiven Messung der Garnmasse konzentriert, ist die Erfindung nicht auf dieses Abtastprinzip beschränkt. Vielmehr können beim erfindungsgemässen Verfahren auch andere Abtastprinzipien - möglicherweise mit anderen Messgrössen - zum Einsatz kommen, z. B. die optische Messung des Garndurchmessers. Auch Kombinationen verschiedener Abtastprinzipien sind möglich.

### BEZUGSZEICHENLISTE

| | |
|---|---|
| 1 | Vorrichtung |
| | |
| 2 | Abtasteinheit |
| 23 | erste Datenleitung |
| | |
| 3 | Auswerteeinheit |
| 34 | zweite Datenleitung |
| | |
| 4 | Ausgabeeinheit |
| | |
| 5 | Eingabeeinheit |
| | |
| 9 | Effektgarn |
| 91, 91' | Steg |
| 92, 92' | Effekt |
| 93,94 | Effektflanken |
| 95 | Effektdach |
| | |
| 100 | Messkurve |
| 101 | Grundpegel |
| 102, 102' | Signalspitze |
| 103, 103', 103* | Effektbeginn |
| 104, 104* | Effektende |
| 105 | lokales Maximum einer Spitze |
| 106 | Dünnstelle neben Effekt |
| | |
| 111-115 | Klassen von Effekten, Effektpopulationen |
| 111.1-113.1 | Teilflächen des Scatterplots |
| | |
| 121 | lokales Maximum, das zu Stegen gehört |
| 122 | lokales Maximum, das zu Effekten gehört |
| | |
| 131 | Verteilung im Spektrogramm |
| 132 | Maximum der Verteilung 121 |
| 133 | Spitze im Spektrogramm |
| | |
| A | Fläche unter der Messkurve |
| B | Farbe Blau |
| H | Häufigkeit eines Messwertes |
| I_{M} | Massenintervall |
| L | Periodenlänge |
| L_{E} | Effektlänge |
| Ls | Steglänge |
| L_{E} + L_{S} | Effektabstand |
| M | Masse pro Längeneinheit |
| M_{E} | Effektgesamtmasse |
| M_{M} | Mittelwert der gemessenen Masse pro Längeneinheit |
| M_{S} | Stegmasse pro Längeneinheit |
| M_{T} | Schwellenwert |
| R | Farbe Rot |
| x | Längskoordinate |
| | |
| ΔM | Massenzunahme eines Effektes |

## Patentansprüche

1. Verfahren zur Charakterisierung von Effektgarn (9), wobei
mindestens eine Eigenschaft des Effektgarns (9) entlang der Längsrichtung (x) des Effektgarns (9) abgetastet wird,
Werte der Abtastung ausgewertet werden und
Resultate der Auswertung ausgegeben werden,
**dadurch gekennzeichnet,**
**dass** mindestens ein Resultat der Auswertung in Form einer grafischen Darstellung ausgegeben wird, wobei
die grafische Darstellung eine Fläche ist, die in einem dreidimensionalen Raum liegt, dessen erste Dimension eine Effektlänge (L_{E}), dessen zweite Dimension eine Massenzunahme (ΔM) oder eine Durchmesserzunahme von Effekten (92) und dessen dritte Dimension eine beobachtete Häufigkeit (H) der entsprechenden Effektcharakteristika (L_{E}, ΔM) angibt, oder
die grafische Darstellung eine Projektion einer solchen Fläche in die durch eine Effektlängenachse und eine Massenzunahmen- oder Durchmesserzunahmenachse aufgespannte Ebene ist.

2. Verfahren nach Anspruch 1, wobei anhand der grafischen Darstellung verschiedene Klassen (111-113) von Effekten (92) identifiziert werden.

3. Verfahren nach Anspruch 2, wobei die identifizierten Klassen (111-113) von Effekten (92) voneinander und/oder von Ausreissern abgegrenzt werden.

4. Verfahren nach einem der vorangehenden Ansprüche, wobei bei der Auswertung aus den Werten der Abtastung eine Messkurve (M(x)) erzeugt und eine Fläche (A(x)) unter der Messkurve (M(x)) berechnet wird.

5. Verfahren nach Anspruch 4, wobei die Fläche (A(x)) zwischen der Messkurve (M(x)) und einer zuvor bestimmten Stegmasse (M_{S}) oder einem zuvor bestimmten Stegdurchmesser berechnet wird.

6. Verfahren nach einem der vorangehenden Ansprüche, wobei die mindestens eine abgetastete Eigenschaft eine Masse (M) und/oder ein Durchmesser des Effektgarns (9) ist.

7. Verfahren nach einem der vorangehenden Ansprüche, wobei Resultate der Auswertung aus der Gruppe von Effektgarnparametern ausgewählt werden, welche eine Stegmasse (M_{S}), einen Stegdurchmesser, eine Steglänge (L_{S}), eine Massenzunahme (ΔM) eines Effektes (92), eine Effektdurchmesserzunahme, einen Effektdurchmesser, eine Effektlänge (L_{E}), eine Effektgesamtmasse (M_{E}), eine mittlere Garnnummer, eine Anzahl Effekte pro Längeneinheit, eine Rapportlänge, eine Unterraportlänge, eine Form und eine Farbe umfasst.

8. Verfahren nach Anspruch 7, wobei jedem Effekt (92, 92') eine Laufnummer zugeordnet und die Laufnummer zusammen mit den Parametern des zugehörigen Effektes (92) gespeichert wird.

9. Verfahren nach Anspruch 7 oder 8, wobei Resultate der Auswertung Minima, Maxima, arithmetische Mittel und/oder Standardabweichungen der Effektgarnparameter und/oder die Anzahl Effekte (92) pro Garnlänge sind.

10. Verfahren nach einem der vorangehenden Ansprüche, wobei die Auswertung eine Glättung oder Idealisierung der Abtastwerte beinhaltet.

11. Verfahren nach Anspruch 10, wobei die Auswertung eine Verknüpfung der geglätteten oder idealisierten Abtastwerte mit den ursprünglichen Abtastwerten beinhaltet.

12. Verfahren nach Anspruch 11, wobei die Verknüpfung eine Differenzbildung oder eine Quotientenbildung ist.

13. Verfahren nach einem der Ansprüche 10-12, wobei die Glättung oder Idealisierung die Annäherung durch gerade Streckenabschnitte beinhaltet und Effekte (92, 92') des Effektgarns (9) als Trapeze, Dreiecke oder Rechtecke angenähert werden.

14. Verfahren nach einem der vorangehenden Ansprüche, wobei die Auswertung eine Filterung der Abtastwerte beinhaltet.

15. Verfahren nach Anspruch 11 oder 12, wobei eine eigene grafische Darstellung für die geglätteten oder idealisierten Abtastwerte und/oder eine eigene grafische Darstellung für die aus der Verknüpfung hervorgegangenen Daten ausgegeben wird.

## Claims

1. A method for the characterization of fancy yam (9), wherein
at least one characteristic of the fancy yam (9) is scanned along the longitudinal direction (x) of the fancy yam (9),
values of the scanning are evaluated and
results of the evaluation are outputted,
**characterized in that**
at least one result of the evaluation is outputted in the form of a graphic representation, wherein
the graphic representation is a surface which lies in three-dimensional space, whose first dimension indicates a slub length (L_{E}), whose second dimension indicates a mass increase (ΔM) or a diameter increase of slubs (92), and those third dimension indicates an observed frequency (H) of the respective slub characteristics (L_{E}, ΔM), or
the graphic representation is a projection of such a surface into the plane spanned by a slub length axis and a mass increase axis or a diameter increase axis.

2. The method according to claim 1, wherein different classes (111-113) of slubs (92) are identified by way of the graphic representation.

3. The method according to claim 2, wherein the identified classes (111-113) of slubs (92) are delimited from one another and/or from outliers.

4. The method according to one of the preceding claims, wherein on evaluation, a measurement curve (M(x)) is produced from the values of the scanning, and an area (A(x)) below the measurement curve (M(x)) is computed.

5. The method according to claim 4, wherein the area (A(x)) between the measurement curve (M(x)) and a previously determined base yam mass (M_{S}) or a previously determined base yam diameter is computed.

6. The method according to one of the preceding claims, wherein the at least one scanned characteristic is a mass (M) and/or a diameter of the fancy yam (9).

7. The method according to one of the preceding claims, wherein results of the evaluation are selected from the group of fancy yam parameters which includes a base yam mass (M_{S}), a base yam diameter, a slub distance (L_{S}), a mass increase (ΔM) of a slub (92), and slub diameter increase, a slub diameter, and slub length (L_{E}), a slub total mass (M_{E}), an average yam count, a number of slubs per length unit, a pattern length, a sub-pattern length, a shape and a color.

8. The method according to of the claim 7, wherein a running number is allocated to each slub (92, 92'), and the running number is stored together with the parameters of the associated slub (92).

9. The method according to claim 7 or 8, wherein results of the evaluation are minima, maxima, arithmetic means and/or standard deviations of the fancy yam parameters and/or the number of slubs (92) per yam length.

10. The method according to one of the preceding claims, wherein the evaluation includes a smoothing or idealization of the scanning values.

11. The method according to claim 10, wherein the evaluation includes a linking of the smoothed or idealized scanning values with the original scanning values.

12. The method according to claim 11, wherein the linking is a difference formation or a quotient formation.

13. The method according to one of claims 10-12, wherein the smoothing or idealization contains the approximation by straight stretch sections and slubs (92, 92') of the fancy yam (9) are approximated as trapeziums, triangles or rectangles.

14. The method according to one of the preceding claims, wherein the evaluation includes a filtering of the scanning values

15. The method according to claim 11 or 12, wherein an individual graphic representation for the smoothed or idealized scanning values and/or an individual graphic representation for the data which has come from the linking is outputted.

## Revendications

1. Procédé pour la caractérisation d'un filé à effets (9), dans lequel au moins une propriété du filé à effets (9) est détectée dans le sens longitudinal (x) du filé à effets (9),
des valeurs de la détection sont analysées et
des résultats de l'analyse sont émis en sortie,
**caractérisé en ce**
**qu'**au moins un résultat de l'analyse est émis en sortie sous forme de représentation graphique,
la représentation graphique étant une aire située dans un espace à trois dimensions dont la première dimension indique une longueur d'effet (LE), la deuxième dimension une augmentation de la masse (ΔM) ou une augmentation du diamètre des effets (92) et la troisième dimension une fréquence observée (H) des caractéristiques de l'effet (LE, ΔM) correspondantes, ou
la représentation graphique étant une projection d'une telle aire dans un plan délimité par un axe de longueur d'effet et un axe d'augmentation de la masse ou d'augmentation du diamètre.

2. Procédé selon la revendication 1, dans lequel différentes classes (111-113) d'effets (92) sont identifiés à l'aide de la représentation graphique.

3. Procédé selon la revendication 2, dans lequel les classes (111-113) identifiées sont délimitées par des effets (92) entre eux et/ou par des valeurs extrêmes.

4. Procédé selon l'une des revendications précédentes, dans lequel lors de l'analyse des valeurs de la détection, une courbe de mesure (M(x)) est créée et une aire (A(x)) sous la courbe de mesure (M(x)) est calculée.

5. Procédé selon la revendication 4, dans lequel l'aire (A(x)) est calculée entre la courbe de mesure (M(x)) et une masse de la section de filé entre deux effets (M_{S}) déterminée au préalable ou un diamètre de la section de filé entre deux effets déterminé au préalable.

6. Procédé selon l'une des revendications précédentes, dans lequel l'au moins une propriété détectée est une masse (M) et/ou un diamètre du filé à effets (9).

7. Procédé selon l'une des revendications précédentes, dans lequel les résultats de l'analyse sont choisis dans le groupe de paramètres du filé à effets comprenant une masse de la section de filé entre deux effets (M_{S}), un diamètre de la section de filé entre deux effets, une longueur de la section de filé entre deux effets (L_{S}), une augmentation de la masse (ΔM) d'un effet (92), une augmentation du diamètre d'un effet, un diamètre d'un effet, une longueur d'un effet (L_{E}), une masse totale d'un effet (M_{E}), un numéro du fil moyen, un nombre d'effets par unité de longueur, une longueur de motif, une longueur de sous-motif, une forme et une couleur.

8. Procédé selon la revendication 7, dans lequel un numéro de passage est attribué à chaque effet (92, 92') et le numéro de passage est enregistré avec les paramètres de l'effet (92) en question.

9. Procédé selon la revendication 7 ou 8, dans lequel les résultats de l'analyse sont des minima, des maxima, des moyennes arithmétiques et/ou des écarts-types des paramètres du filé à effets et/ou le nombre d'effets (92) par longueur de fil.

10. Procédé selon l'une des revendications précédentes, dans lequel l'analyse comprend un lissage ou une idéalisation des valeurs de détection.

11. Procédé selon la revendication 10, dans lequel l'analyse comprend une association des valeurs de détection lissées ou idéalisées aux valeurs de détection d'origine.

12. Procédé selon la revendication 11, dans lequel l'association est un calcul de différence ou un calcul de quotient.

13. Procédé selon l'une des revendications 10 à 12, dans lequel le lissage ou l'idéalisation comprend une approximation par des tronçons de ligne droits et les effets (92, 92') du filé à effets (9) sont approchés sous forme de trapèzes, de triangles ou de rectangles.

14. Procédé selon l'une des revendications précédentes, dans lequel l'analyse comprend un filtrage des valeurs de détection.

15. Procédé selon la revendication 11 ou 12, dans lequel une représentation graphique propre des valeurs de détection lissées ou idéalisées et/ou une représentation graphique propre des données issues de l'association sont émises en sortie.
